# EUROPEAN PATENT APPLICATION

(11) **EP 3 805 357 A2**
(43) Date of publication of application: **14.04.2021**
(21) Application number: 20196931.8
(22) Date of filing: 18.09.2020
(51) Int. Cl.: C12M 1/00, C12M 1/24, C12M 1/02, C12M 3/04, C12M 1/34

(54) **CELL CULTURE FLASKS, SENSOR INSERTS, AND SYSTEMS AND METHODS COMPRISING THE SAME**

(30) Priority: 20.09.2019 US 201962903250 P; 09.04.2020 US 202063007601 P
(71) Applicant: Scientific Industries, Inc., Bohemia, New York 11716 (US)
(72) Inventor: Cremonese, Joseph G., Greensburg, Pennsylvania 15601 (US)
(74) Representative: Fritzsche, Thomas

(57) **Abstract**

Embodiments disclosed herein relate to novel flasks, methods and systems employing said novel flasks, and sensors for use within said flasks and systems. In some embodiments, flasks and systems described herein are used to culture cells and/or detect changes in pH levels or dissolved oxygen and/or carbon dioxide levels in a cell culture sample resulting from the growth of living cells contained within a reaction vessel such as a flask. The devices may be used to detect changes in pH and dissolved oxygen levels in a liquid contained in the reaction vessel due to growth of living cells.

## Description

### CROSS REFERENCE TO RELATED APPLICATIONS

This application claims the benefit of U.S. Provisional Application Ser. No. 63/007,601 filed on April 9, 2020, and entitled "Cell Culture Flasks, Sensor Inserts, And Systems And Methods Comprising The Same", which claims priority from U.S. Provisional Application Ser. No. 62/903,250, filed on September 20, 2019 and entitled "Reaction Vessel and Extendable Sensor Stick", both of which are incorporated herein by reference in their entirety.

### BACKGROUND

### Field of the Invention

Embodiments disclosed herein relate to novel flasks, methods and systems employing said novel flasks, and sensors for use within said flasks and systems. In some embodiments, flasks and systems described herein are used to culture cells and/or detect changes in pH levels or dissolved oxygen and/or carbon dioxide levels in a cell culture sample resulting from the growth of living cells contained within a reaction vessel such as a flask.

Shake flasks, conical flasks, and Erlenmeyer flasks are commonly employed in laboratories for cell cultures, including plant and microbial cell cultures.

Shake flasks have flat bottoms and sloping sides and are generally made of glass, plastic or metal. The width of the flask opening can vary and is typically fitted with a porous cap or plug closure. Gases can be exchanged through the closure without the introduction of contaminating substances or organisms.

In a typical culturing procedure, flasks containing culture broth are placed on shaking tables or in incubator-shakers. The shaking table or incubator's movement causes mixing and mass transfer inside the flask. Shaking can be performed using either an orbital motion (also called "rotary shaking" or "gyratory shaking"), or using a linear reciprocating (*i.e*., a back-and-forth) motion.

Orbital shakers are becoming more common than reciprocating shakers.

The first obstacle encountered in the delivery of oxygen to shake-flask cultures is the flask closure. A variety of porous materials may be used to stopper shake flasks, such as cotton, cotton wool bound with cheesecloth, and silicone sponge.

The rate of oxygen transfer through the closure depends on the diffusion coefficient for oxygen in the stopper material, the width of the flask neck opening, and the stopper depth.

Gas (e.g., oxygen) transfer becomes significantly more difficult if the stopper has been wetted. Additionally, wetting the flask stopper increases the risk of culture contamination, which is especially a problem when flasks are vigorously shaken.

The flask closure affects the composition of gas in the headspace of the flask. In addition to impeding oxygen transfer into the culture, the closure prevents rapid escape of carbon dioxide and other gases generated by the cultured cells. Volatile substances such as ethanol may also build up in the gas phase.

Although several types of flask stoppers have been investigated for their oxygen transfer characteristics, it is unclear which closure gives the best results. In some cases, such as with large flasks and/or if the liquid is shaken very vigorously, the flask closure becomes the dominant resistance for oxygen transfer to the cells.

The culture broth in shake flasks is generally supplied with oxygen by surface aeration from the gas atmosphere inside the flask. Within the flask, the main resistance to oxygen transfer is the liquid-phase boundary layer at the gas-liquid interface.

Oxygen transfer in shake flasks is dependent on several variables, including flask shape, flask size, surface properties of the flask walls (*i.e*., whether hydrophilic or hydrophobic), shaking speed, flask displacement during shaking, liquid volume, and liquid properties (*e.g*., viscosity).

Surface-to-volume ratios decrease with increasing liquid volume, thus flask culture and surface aeration are practical only at relatively small scales. Therefore, for some cultures, the maximum rate of oxygen transfer in shake flasks is insufficient to meet the cellular oxygen demand. In these cases, shake flasks offer insufficient oxygen supply and the culture performance is limited by oxygen transfer.

Indeed, experiments performed by Zhu et al provide evidence that the surface-to-volume ratio of a flask culture has an effect on the oxygen transfer rate (Zhu, L., Han, W., Song, B. and Wang, Z. (2018), Characterizing the fluid dynamics in the flow fields of cylindrical orbitally shaken bioreactors with different geometry sizes. Eng. Life Sci., 18: 570-578). These experiments examined how the geometry of cylindrical orbitally shaken bioreactors (OSR) affected their oxygen transfer rates. The fluid dynamics of each OSR were calculated using computational models and validated through experimental methods. Overall, the results from the experiments indicated that by increasing the contact area between the liquid phase of the culture and the surface area of the inner wall of the cylindrical OSR, an increase in the efficiency of oxygen transfer could occur in the culture.

Therefore, some of the objectives of the present disclosure are to provide flasks with improved designs, systems described herein comprising said flasks, and methods described herein of using said flasks as a means of culturing cells wherein the problem of inhibiting gas (e.g., air or oxygen) flow is eliminated. Flasks, systems, and methods described herein permit air/oxygen to freely flow regardless of the size of the flask neck or the type of material used in the flask stopper.

A further object of flasks, systems and methods described herein is to enable the use of a faster rotation without the concern of wetting a flask stopper.

### SUMMARY

Described herein are various, non-limiting embodiments of a cell culture flask comprising a lower portion and an upper portion, said lower portion comprising a flat bottom and a curved wall portion curving upward to a vertical or substantially vertical wall portion extending upward to the upper portion, said upper portion comprising an angled wall, angled upward to a flask neck or mouth.

Described herein are various, non-limiting embodiments of an inverted cell culture flask system for culturing cells comprising a flask oriented in an inverted fashion, a vacuum pump, a stopper, a light scattering source, a detector system, and at least one optical sensor operably connected to the detector system.

Additionally described herein are various, non-limiting embodiments of a method of culturing cells comprising culturing cells in a flask oriented in an inverted fashion, wherein the flask comprises a stopper, the stopper comprising a light scattering source and one or more optical sensors, operably connected to a detector system.

Also described herein are various, non-limiting embodiments of a sensor insert comprising: at or proximate to a first end, a hollow or substantially hollow tube portion, a space for housing one or more carbon dioxide absorbent substances, an extension length, and at a second end, a sensor tab configured to hold one or more sensor patches.

Furthermore, described herein are various, non-limiting embodiments of a flask stopper comprising a light scattering source and one or more optical sensors.

While these potential advantages are made possible by technical solutions offered herein, they are not required to be achieved. Embodiments of the presently disclosed system and method can be implemented to achieve technical advantages, whether or not these potential advantages, individually or in combination, are sought or achieved.

Further features, aspects, objects, advantages, and possible applications of the present invention will become apparent from a study of the exemplary embodiments and examples described below, in combination with the Figures, and the appended claims.

### BRIEF DESCRIPTION OF THE DRAWINGS

The above and other objects, aspects, features, advantages and possible applications of the present invention will be more apparent from the following more particular description thereof, presented in conjunction with the following drawings, in which:
FIGs. 1A-1H depict embodiments of an exemplary BioCoaster detector system described herein.
FIG. 2A depicts an embodiment of a sensor insert described herein.
FIG. 2B depicts an embodiment of a sensor insert described herein from which the hollow tube portion has been detached from the remainder of the sensor insert.
FIG. 2C depicts an embodiment of a sensor insert described herein which has been inserted into a vertically arranged reaction vessel.
FIG. 2D depicts an embodiment of an angled-shaped sensor insert described herein. FIG. 2E depicts an embodiment of the angled-shaped sensor strip described herein within a horizontally arranged reaction vessel.
FIG. 2F depicts an embodiment of a reader unit juxtaposed with an embodiment of a vertically aligned reaction vessel in which an L-shaped sensor insert described herein has been inserted.
FIG. 2G depicts an embodiment of a reader unit placed underneath an embodiment of a vertically aligned reaction vessel in which an L-shaped sensor insert described herein has been inserted.
FIG. 2H depicts an embodiment of a reader unit placed underneath an embodiment of a horizontally aligned reaction vessel in which an angled-shaped sensor insert described herein has been inserted.
FIG. 3A depicts an embodiment of an exemplary wine-glass shaped flask described herein.
FIG. 3B depicts an embodiment of an exemplary wine-glass shaped flask described herein, situated on an embodiment of a BioCoaster described herein.
FIG. 4 depicts an embodiment of an exemplary cell culture system comprising a cell culture flask in an inverted orientation.
FIG. 5A depicts an embodiment of the inverted-flask system wherein the inverted flask is attached to a swivel adapter.
FIG. 5B depicts an embodiment of the inverted-flask system wherein the inverted flask is attached to a swivel adapter and has begun spinning to where the solution tilts to the left side of the flask.
FIG. 5C depicts an embodiment of the inverted-flask system wherein the inverted flask is attached to a swivel adapter and continues spinning to where the solution tilts to the right side of the flask.
FIG. 5D depicts an embodiment of the inverted-flask system wherein the inverted flask is attached to a swivel adapter and is spun at a speed of 100 rpm.

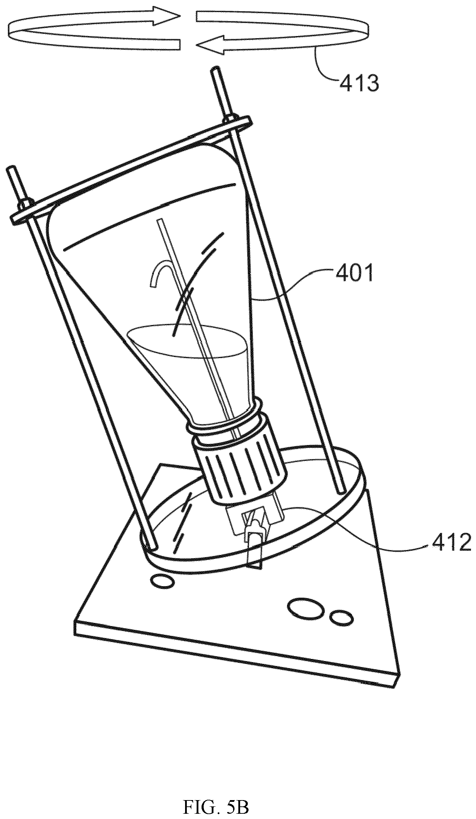

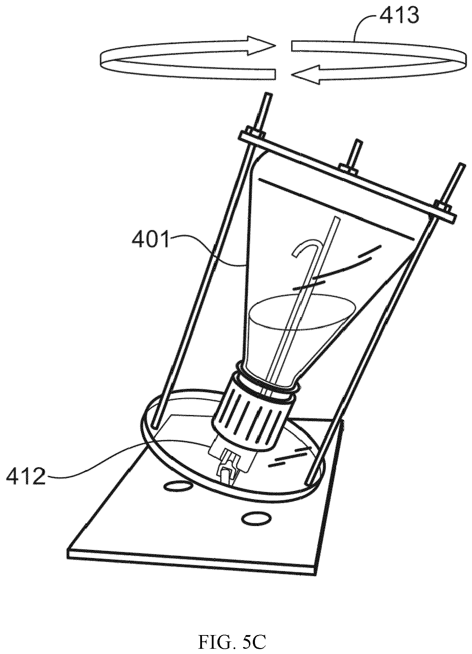

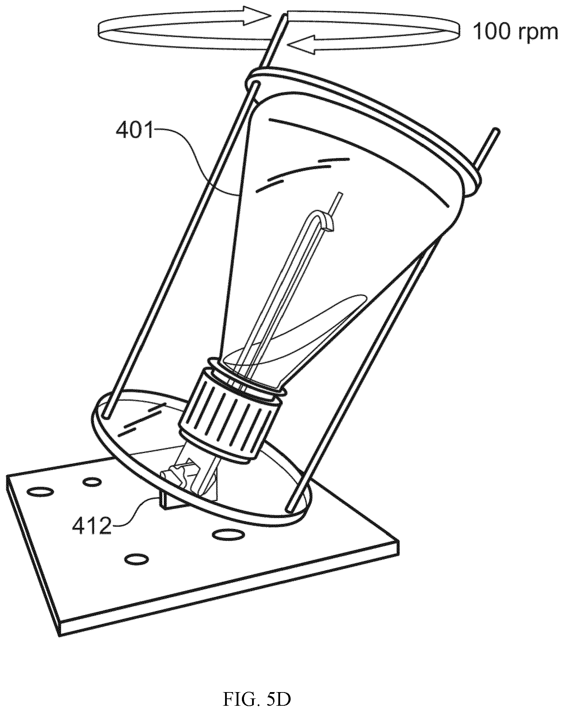

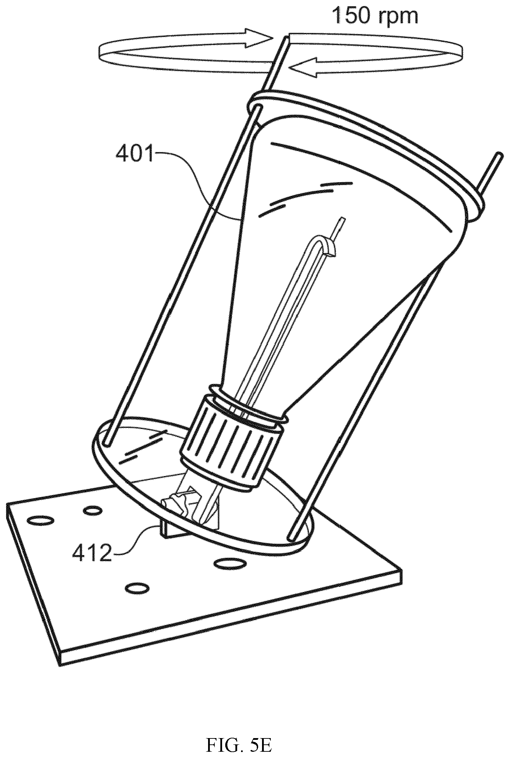

FIG. 5D depicts an embodiment of the inverted-flask system wherein the inverted flask is attached to a swivel adapter and is spun at a speed of 150 rpm.

### DETAILED DESCRIPTION OF THE INVENTION

The following description is of an embodiment presently contemplated for carrying out the present invention. This description is not to be taken in a limiting sense, but is made merely for the purpose of describing the general principles and features of the present invention. The scope of the present invention should be determined with reference to the claims.

### Exemplary Sensor Inserts

Also described herein are sensor inserts for use in cell culturing methods and systems described herein.

Embodiments can relate to a cell culture incubator system having a sensor insert (or "sensored strip") configured to be placed, in exemplary embodiments, within a flask described herein and/or used in conjunction or combination with a shake flask system described herein or, in other embodiments, any suitable reaction vessel selected by a person of skill in the art. The sensor insert can be configured so that when inserted, the sensor is positioned within media covering the cells. A reader (e.g., a BioCoaster, described in detail below) is placed outside but adjacent the vessel to read the sensor so as to detect changes in dissolved O₂ and pH. The sensor insert is used to determine if the incubator environment is has too much CO₂ and is therefore trending towards hypoxia and/or acidity, or has too much dissolved O₂ and is therefore trending toward oxygen toxicity of the cell nutrient media. Other conditions that can be determined may include the onset of apoptosis, growth toward equilibrium, deviations from equilibrium, etc.

Exemplary embodiments of sensor inserts for use in embodiments described herein are provided in U.S Provisional Application No. 62/896,211, filed on September 5, 2019. The entire contents of U.S. Provisional Application No. 62/896,211 are incorporated herein by reference.

In an aspect, the sensor insert can be a rigid or semi-rigid member (e.g., polymer, plastic, glass, metal, etc.) configured to be removably inserted into the reaction vessel. In certain embodiments, the sensor insert is made from clear (e.g., transparent or translucent) plastic material. The sensor insert can have at least one sensor disposed or attached to a portion of the strip.

The shape and configuration of the strip, as well as the placement of the sensor thereon, can be such that the sensor resides within the media when the sensor insert is inserted within the reaction vessel. For instance, the sensor insert can have a first end and a second end, with an elongated shaft extending between the first end and the second end. The elongated shaft can be straight, bent, angled, etc. so that after the second end (e.g., a distal end) is inserted into the reaction vessel, it rests on a bottom portion of the reaction vessel. It is contemplated for the sensor to be located at or near the second end, but it can be located at any portion of the strip. The details of the sensor and the reader will be explained later, but it is contemplated for light to be imparted on the sensor and for the sensor to emit light based on the levels of dissolved O₂ and/or pH of the media within which it is submerged. Thus, the strip portion where the sensor is located should be transparent to the anticipated light that will be imparted to the sensor and emitted therefrom.

In an aspect, a sensor insert described herein may be L-shaped or substantially L-shaped, or angled-shape or substantially angled-shaped, or linear in shape or substantially linear in shape.

Additionally, a sensor insert described herein comprises a portion extending from the tube portion

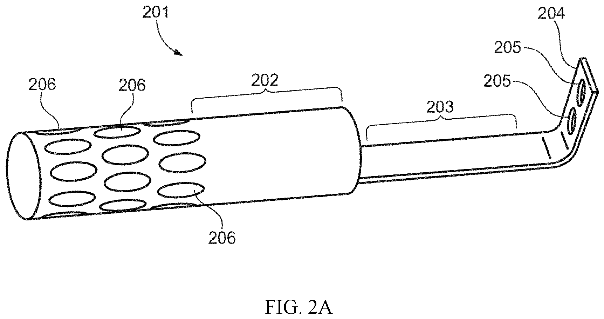

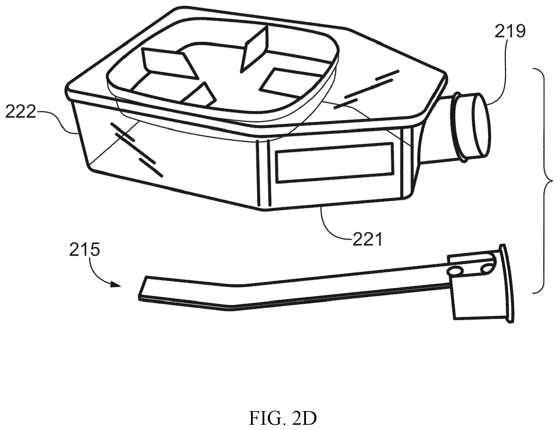

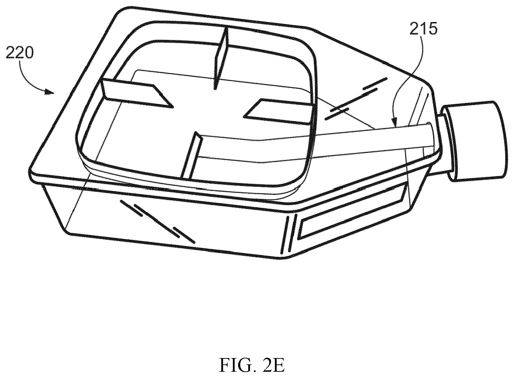

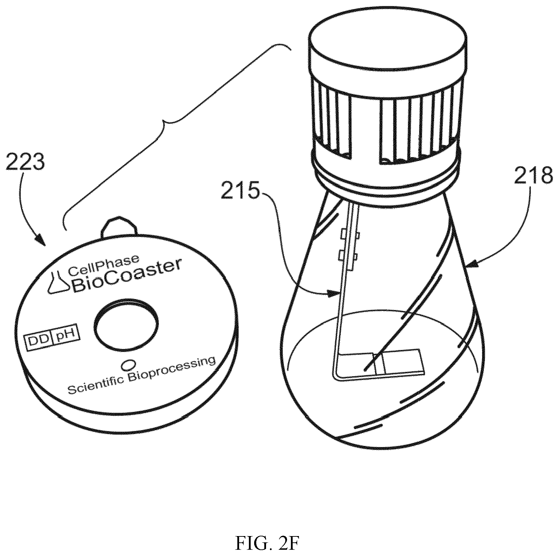

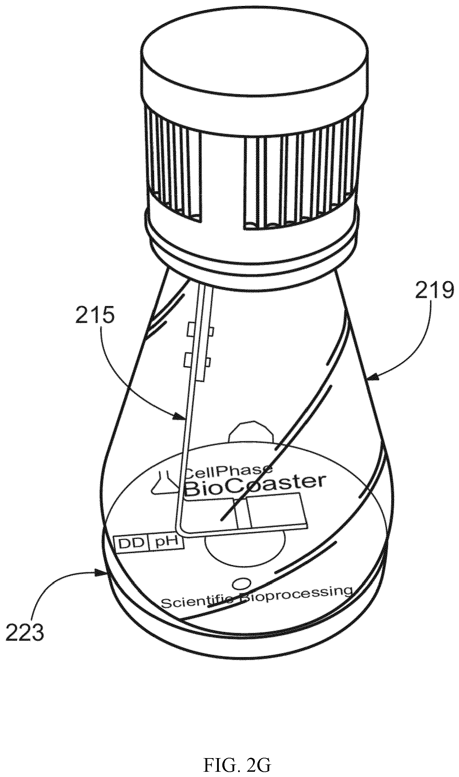

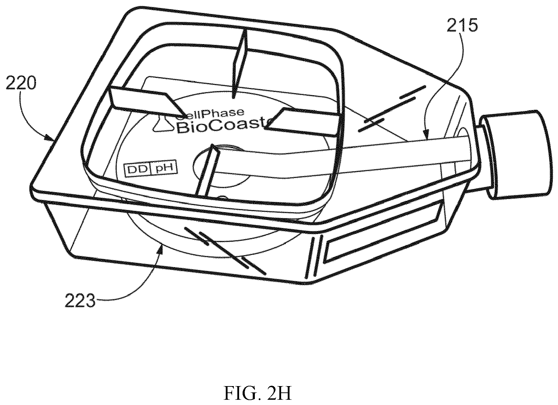

Referring to FIG. 2A, in a typical embodiment, a sensor insert 200 described herein is an L-shaped extension having a hollow tube portion 201 located at or proximally to a first end, optionally comprising a space for housing absorbents 202, and having an extension length 203 which is adjustable to permit insertion of the sensor insert into differently sized flasks. In a typical embodiment, a sensor insert 200 described herein has a sensor tab 204 at a second end, said sensor tab 204 being configured to hold sensor patches 205. Additionally, the sensor insert 200 may optionally have vents 206 located in the hollow tube portion 201, said vents 206 permitting carbon dioxide to enter an inner tube space (not pictured) of the hollow tube portion 201 from the flask head-space.

In an aspect, a preferred embodiment of the sensor insert described herein traps carbon dioxide.

In a preferred embodiment, the sensor insert comprises a space for housing carbon dioxide absorbents.

In a further preferred embodiment, the sensor insert comprises both a space for housing absorbents carbon dioxide absorbents and vents.

In some embodiments, the tube portion of the sensor insert may contain one or more carbon dioxide absorbents, such as hydroxides, carbonates, bicarbonates, chlorides and oxides of alkalai metals or alkaline earth metals. For example, and without limitation, suitable carbon dioxide absorbents include sodium hydroxide, sodium carbonate, sodium bicarbonate, potassium hydroxide, lithium hydroxide, lithium chloride, calcium oxide, calcium carbonate, calcium hydroxide, soda lime, activated carbon, and other known carbon dioxide absorbents.

In certain embodiments, the tube portion of the sensor insert comprises a space therein for holding pellets of a carbon dioxide absorbent.

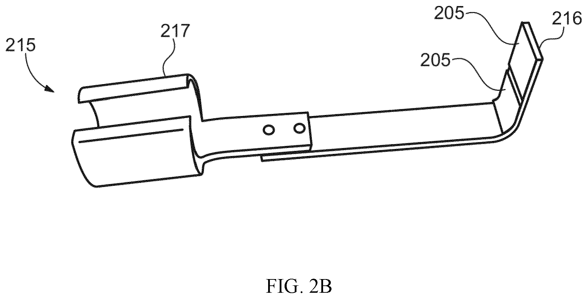

In certain embodiments, the hollow tube portion 201, optionally connected to or otherwise housing a space for housing absorbents 202, is detachable from the sensor insert 200. FIG. 2B depicts an exemplary embodiment of a sensor insert 215 from which the hollow tube portion 201, optionally connected to or otherwise housing a space for housing absorbents 202, has been detached from the remainder of the sensor insert 215.

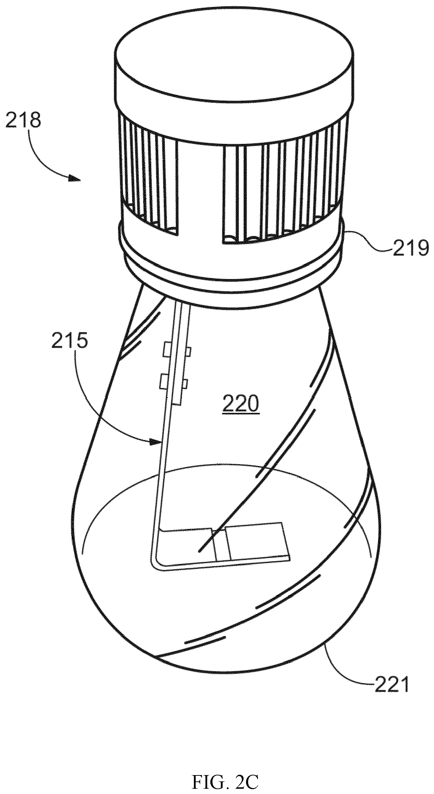

FIGs. 2C-2H depict further embodiments of sensor inserts described herein.

In certain embodiments, a sensor insert described herein comprises a hollow or substantially hollow tube, wherein the tube has one or more vents. In another aspect, the vents located on the tube portion of the sensor insert allow carbon dioxide to enter an inner tube space from the flask head-space.

In an aspect, the sensor patches 205 are to be aligned with a separate, external sensor-reading device. In certain embodiments, the sensor-reading device is a BioCoaster. Embodiments using different sensor-reading devices are hereby contemplated, so long as said sensor-reading devices are compatible with the sensor-spots 205 housed on the sensor tab 204.

Various embodiments of the present disclosure can be described in terms of the example sensor insert described herein; however, other embodiments of the sensor insert, along with other flask and coaster (e.g., BioCoaster) embodiments, can be used.

As a non-limiting example, a sensor insert 215 described herein can be an elongated shaft with a second end 216 having a sensor disposed thereon and a first end 217 having a stopper disposed thereon. The elongated shaft can extend straight from the first end 217 and form an L-shape that leads to the second end 216. Such an L-shape sensor insert can be used for a vertically arranged reaction vessel 218. The L-shaped second end 216 can be inserted through the open mouth 219 of the vertically arranged reaction vessel 218 until the engagement of the stopper at the first end 217 with the vessel mouth 219 suspends the sensor insert second end 216 in the cavity portion 220 of the reaction vessel so that the second end 216 resides at the bottom of the reaction vessel 221 or just above the bottom of the reaction vessel. The L-shape allows the second end 216 (and the sensor(s)) to be suspended so that it is parallel with the reaction vessel bottom 221, thereby allowing it to be submerged in the media. As another non-limiting example, the sensor insert 215 can be an elongated shaft with a second end 216 having a sensor disposed thereon and a first end 217 having a stopper disposed thereon. The elongated shaft can extend straight from the first end 217 and form an angled-shape that leads to the second end 216. Such an angled-shape sensor insert can be used for a horizontally arranged flask or reaction vessel 222. The angled-shaped second end 216 can be inserted through the open mouth 219 of the horizontally arranged reaction vessel 222 until the engagement of the stopper at the first end 217 with the vessel mouth 219 suspends the sensor insert second end 216 in the cavity portion 220 of the reaction vessel so that the second end 216 resides at the bottom of the reaction vessel 221 or just above the bottom of the reaction vessel. The angled-shape allows the second end 216 (and the sensor) to be suspended so that it is parallel with the reaction vessel bottom 221, thereby allowing it to be submerged in the media.

In an aspect, in operation, the sensor insert 215 can be sterilized and packaged into a sealed container or bag. When it is desired to have the dissolved O₂ and/or CO₂ level(s) of an incubator environment of a reaction vessel measured, a user can un-package the already sterilized sensor and insert it within the reaction vessel (obviating the need to buy expensive vessels with sensors already attached and obviating the need to sterilize the sensor before use). A reader 223 can be placed adjacent, but outside the reaction vessel. The reader 223 can be used to accurately detect the dissolved O₂ or CO₂ (or pH levels) by receiving and analyzing light being emitted from the sensor 215. The inventive system can provide in a quick, accurate, quantitative measurement of dissolved O₂ or CO₂ in an efficient and cost-effective manner. It should be noted that the inventive system can also obviate the need to properly align a reader with a sensor that has already been attached to the reaction vessel, as is required for existing systems.

In an aspect, a sensor insert described herein may house or otherwise comprise one or more sensors. In an aspect, a sensor insert described herein may house one or more sensors (i.e., sensor patches) for measuring dissolved oxygen levels, one or more sensors for measuring pH, or a combination of one or more sensors for measuring dissolved oxygen levels and one or more sensors for measuring pH.

An example of a pH sensor patch can include impregnating a material with any known ratiometric pH sensitive dye, such as 1-hydroxypyrene-3,5,7-sulfonic acid (HPTS). A sterilized solution of the dye can be directly introduced into a bioreactor media and detected via fluorescence. Fluorescence detection can be determined using front face geometry. For example, HPTS has two excitation peaks-400 and 450 nm. When excited at either 400 or 450 nm, HPTS can emit light at approximately 520 nm. The longer excitation peak can be excited using a blue LED (460 nm), for example, and the shorter excitation peak can be excited using an UV LED (375 nm), for example. The intensity ratio of the 520 nm fluorescence emissions from excitation at each of the two excitation peaks can be affected by the pH of the media. Thus, the pH can be calibrated by measuring the intensity ratio of the 520 nm fluorescence emissions at each of the two excitation peaks as the pH changes. pH can be optionally verified on a benchtop pH meter. This ratiometric approach may avoid interference from turbidity changes and provides accurate measurements of pH. The pH patch can be structured to generate a ratio-metric response. For example, the chemicals impregnated into the pH patch may be excited by two different, but close, excitation light beams (e.g., different with respect to wavelengths). This can cause generation of two different emission light beams (e.g., each having a different wavelength). Each wavelength of the different emitted light beams can differ depending on changes in pH levels the pH patch is exposed to. The ratio between wavelengths of the different emission light beams can be used as an indicator of the pH level of a growth media in the reaction vessel.

The dissolved oxygen (DO) patch can be a fluorescent oxygen-sensing patch, which can be structured to use oxygen as a quenching agent to quench a chemical response of chemicals impregnated into the DO patch while exposed to a presence of oxygen. The DO patch can be further structured to radiate emitted light when excited by a single excitation light beam. The DO patch can be further structured to generate an emitted light beam as a function of the chemical response. In some embodiments, the DO patch can be structured such that the more oxygen that is present in the environment within which the DO patch is located, the less the chemical response occurs. This may lead to a weaker emission light beam signal. Thus, the less oxygen that is present, the greater the chemical response occurs. This may lead to a stronger emission light beam signal generated by the system. Thus, the lower the levels of DO within a culture sample, the stronger the signal that can be detected from the emitted light. Generally, oxygen content of the growth media in a culture growth bioprocess is less than that of ambient air. Therefore, enabling generation of strong signals in environments where oxygen content is within a range from greater than 0% to 21% can be beneficial for cell culture monitoring.

In certain embodiments, the sensor patch(es) can be mediated by changes in pH levels and/or changes in DO levels in growth media that support growth of cells within the reaction vessel. Detected emitted light radiating from the sensor patch can be captured as signals and digitized by the detector. The digitized signals can be transmitted as reaction vessel data to the computer device. The computer device can be located within and/or outside of a bio-safety laboratory. With reaction vessel data, the computer device can be programmed to calculate fluorescence lifetimes and decay rates associated with oxygen concentration. This can be done to calculate DO concentration within a growth media of cell culture sample.

The sensor patch can be a fluorescent-based optical patch. In one embodiment, the sensor patch can include a substrate with a polymeric backing. The substrate can be filter paper, which may be a monomer (e.g., methyl methacrylate) or cellulose-based filter paper. In some embodiments, the backing can include a silicon-based backing. The backings and/or the substrate can be configured to facilitate quick absorption of the excitation light beams and quick radiation of emitted light beams. One way to achieve this is by combining at least two fluorescent dyes in a monomer while depositing a measured amount of the same onto a surface of the strip. The measured amount can also be polymerized. It should be noted that the water solubility of polymers may vary, and thus polymer selection for the backing may be difficult. It should be further noted that the activity of chemical constituents of polymers can vary widely. Because of this potential variability, pre-calibration of the fluorescent response of a polymetric spot can be difficult to perform. However, using filter paper as a matrix for the reactive chemicals and dyes can facilitate easier calibration. Using filter paper as a matrix for the reactive chemicals and dyes can further increase the rate at which a response may be generated.

In one exemplary embodiment, a sterile optical sensor patch can be placed aseptically on the sensor insert. The back of the sensor patch can be coated with an adhesive for adhering it to the sensor insert. The adhesive can be a biocompatible adhesive. For example, silicone based adhesives with no support binders have been shown to be biocompatible. Some embodiments of the flask or reaction vessel can have an optical window. With such embodiments, the sensor patch can be placed adjacent the flask or reaction vessel optical window and/or at a position covering the flask or reaction vessel optical window. In one embodiment, the sensor patch can be placed at a position subtending the optical window.

Other examples of pH and DO sensor patches can be based on techniques disclosed in U.S. Patent No. 6,673,532, filed August 14, 2001, titled "Bioreactor and Bioprocessing Technique," which is incorporated herein by reference in its entirety.

### Exemplary Detection Systems (BioCoasters)

Also described herein are calibrated fluorometers (or "mini-fluorometer" or "BioCoaster" or, generally, "coaster") for use in cell culturing methods and systems described herein. Each sensor patch or set of sensor patches, calibrated to a mini-fluorometer (i.e., a "BioCoaster") can be used to generate a non-interchangeable system for reading. The reading can be for pH and dissolved oxygen, for example. The detection system can further include a beam combiner assembly, which may be designed so that specific optical filters and LED's may be used. Use of the specific optical filters and LED's can be such that each one is identical in response. Therefore, each beam combiner assembly can be interchangeable.

The beam combiner assembly, including all required optical filters and LED's, may be installed on the digital sensor board to generate an embodiment of the mini-fluorometer. With the configuration of the detection system, it may not be necessary to calibrate each optical sensor patch to a specific BioCoaster each time the system is used. Instead, because of the design of the beam combiner assembly and in conjunction with the digital sensor board, optical sensor patches may be calibrated to a "GOLD COASTER" and the calibration will work with each and every other coaster produced using the beam combiner assembly. In some embodiments, the beam combiner assembly can include the detector. Both the beam combiner assembly and the detector can be housed within a coaster shaped casing upon which the reaction vessel may be placed for monitoring.

In some embodiments, the beam combiner assembly can further include at least one illumination source, a beam combiner, a first filter/mirror arrangement, a second filter/mirror arrangement, and the detector. In some embodiments, the at least one excitation light beam can be caused to be sent through the beam combiner to be combined with another excitation light beam. The combined excitation light beam may be collimated and made to be coaxial. The combined excitation light beam may be further directed to be incident on the at least one patch within the reaction vessel. In some embodiments, the combined excitation light beam can induce the emitted light from the at least one patch due to induced fluorescence. In some embodiments, the emitted light being incident upon both the first filter/mirror arrangement and the second filter/mirror arrangement may be detected by the detector. In some embodiments, the beam combiner assembly can be housed within a circuit board block. A filter of at least one of the first filter/mirror arrangement and the second filter/mirror arrangement can be seated within a base of the circuit board block. A mirror of at least one of the first filter/mirror arrangement and the second filter/mirror arrangement can be shifted with respect to its associated filter.

Embodiments can relate to a cell culture incubator system having BioCoaster configured to be placed, in exemplary embodiments, under a flask described herein and/or used in conjunction or combination with a shake flask system described herein or, in other embodiments, any suitable reaction vessel selected by a person of skill in the art. The BioCoaster can be configured so that when a sensor insert is inserted into a flask or reaction vessel, the sensor is positioned within media covering the cells and above or adjacent to the BioCoaster. The BioCoaster is placed outside but adjacent the vessel to read the sensor so as to detect changes in dissolved O₂ and pH. Other conditions that can be determined may include the onset of apoptosis, growth toward equilibrium, deviations from equilibrium, etc.

Exemplary embodiments of detectors, e.g. BioCoasters, for use in embodiments described herein are provided in U.S Patent No. 10,379,047, issued on August 13, 2019. The entire contents of U.S. Patent No. 10,379,047 are incorporated herein by reference.

Chemicals of the sensor patches can be configured to fluoresce with a first expected wavelength of light as an indicator of a threshold level of DO in the culture sample and with a second expected wavelength of light as an indicator of a threshold level of pH in the culture sample. For example, it can be expected that a threshold level of DO within the culture sample would generate emitted light having a wavelength corresponding to red visible light when the patch within an environment of that culture sample is caused to fluoresce. It can be further expected that a threshold level of pH within the culture sample would generate emitted light having a wavelength corresponding to green visible light when the patch within an environment of that culture sample is caused to fluoresce.

In some embodiments, an expected offset between wavelengths of emitted light associated with DO threshold levels and pH threshold levels can be determined. For example, as emitted wavelength increases from green to red, a waveform phase offset occurs in the sine wave period because of a slight but accumulating system delay. The phase offset can be measured as an angle, considering that a complete sine wave period is 360 degrees with 90 degrees being the positive peak and 270 degrees being the negative peak. The phase offset angle can be stored in a memory of a computer device. This phase offset angle can be compared to a previously recorded positive peak that occurs when light passing through a red reflector (in the absence of a patch and absence of a culture sample) and detected. In other words, the light detected when the red reflector is in place can be used as the expected phase offset and stored in a computer device to be compared to when light emitted from a patch is detected. If a phase offset occurs as a result of the emission of a fluorescent signal, it may indicate that the emission frequency is at the red end of the calibrated range, and therefore must be emitting from a dissolved oxygen patch that responds to excitation by emitting at approximately 610 nm in wavelength. Embodiments of the detection system can be used to eliminate the need for an operator of the system to identify the sensor patch. Embodiments of the detection system may also, or alternatively, be used to eliminate manually determining which patch, or type of patch, is being used when measuring DO and/or pH. Thus, an expected difference in wavelength between visible red light and visible green light can be used by an embodiment of the detection system to automatically ascertain which patch (a DO patch or a pH patch) is generating emitted light due to induced fluorescence. In some embodiments, the detection system can automatically determine which patch is being used and can adjust accordingly. Such techniques can further eliminate manually entering patch data corresponding to a type of patch into a culture monitoring system, which can save time and reduce error.

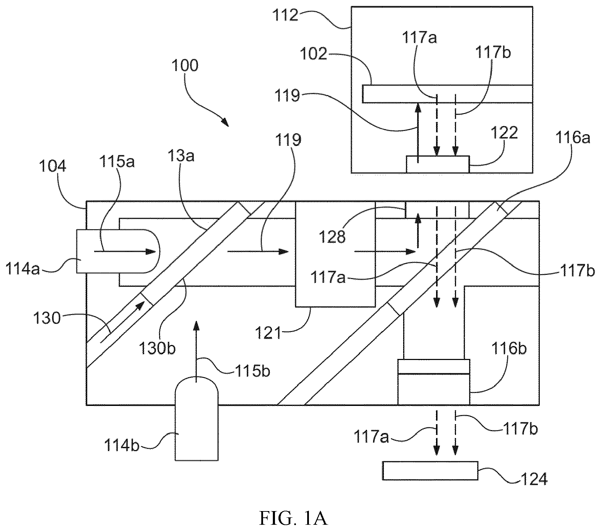

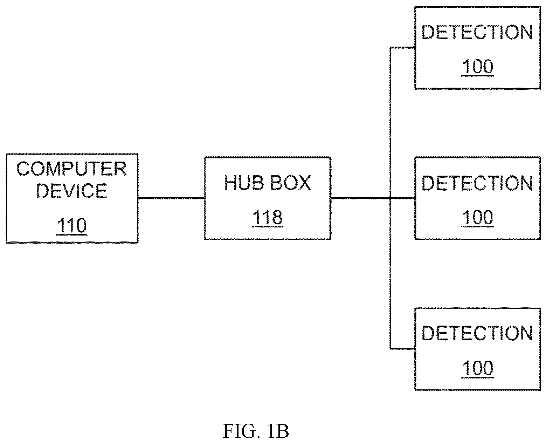

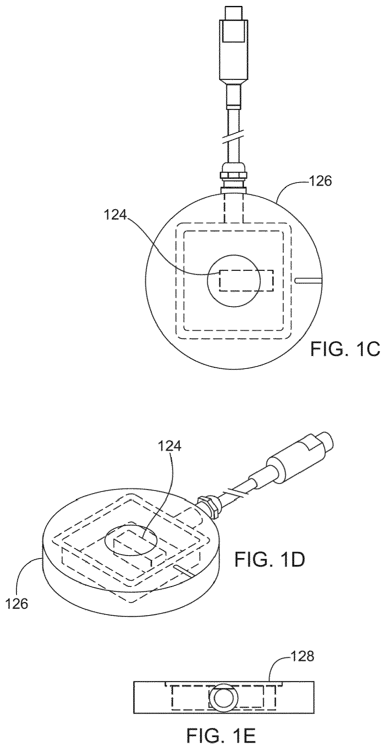

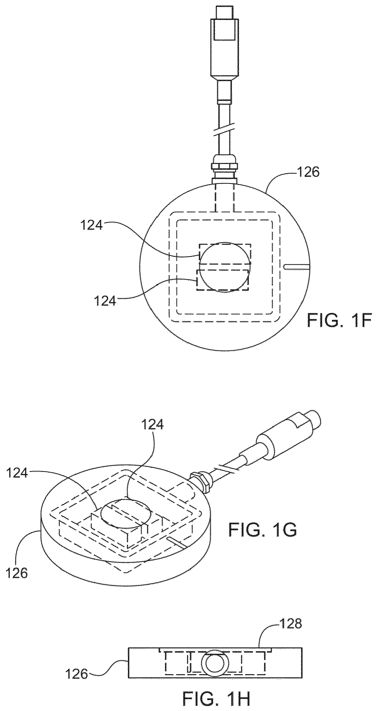

Referring to FIGs. 1A and 1B, a typical, non-limiting embodiment of a detection system 100 described herein can include a fluorescent-based patch 102 and a beam combiner assembly 104. Some embodiments can include at least one computer device 110 in connection with the detection system 100 (see FIG. 1B). The computer device 110 can be programmed to run software generating user interfaces 108 that may be displayed via at least one computer device 110. The computer device 110 may be part of a computer system. Some embodiments include use of a patch 102. The patch 102 may be can be placed into a reaction vessel 112 containing a culture sample to be monitored. The beam combiner assembly 104 can include at least one illumination source 114a, 114b, optical filters/mirrors 116a, 116b, and/or other optoelectronics, which can be used to generate excitation light beams 115a, 115b and detect emitted light 117a, 117b that may be induced by fluorescence. A hub box 118 can be used to communicatively and operably associate the computer device 110 to at least one beam combiner assembly 104. The hub box 118 can be a data acquisition card, for example. Use of a hub box 118 can facilitate data transmission between the computer device 110 and each beam combiner assembly 104. In some embodiments, at least one hub box 118 can be used to acquire data from a plurality of reaction vessels 112. In further embodiments, the hub box 118 can be used to determine a life of the patch 102, which may be defined as the time frame by which the patch 102 can effectively fluorescence. This can be done by acquisitioning code data from the patch 102. For instance, the code can be manufacturing data, for example, that may be transmitted to the computer device 110 to calculate expiration times. Other data, such as an expected offset of a patch 102 can be encoded within the code that is associated with the patch 102. This code can include information such as a date of manufacture of the patch 102, a date at which the expected offset was determined, a date the patch 102 was packaged, etc. Chemicals within the patch 102 can photo-bleach after a statistically predetermined use has elapsed (e.g., imparting excitation light beams on the patch 102 every fifteen seconds for ninety days can cause chemicals impregnated into the patch 102 to fail to effectively fluoresce and radiate emitted light), and thus the code can include an expected expiration date based on the date of manufacture. In another embodiment, the computer device 110 can calculate the expected expiration date based on such data. The combined light beam 119 can be directed toward the first filter/mirror arrangement 116a, which may be a dichroic filter/mirror. The combined light beam can be further sharpened before being incident upon the first filter/mirror arrangement 116a by a beam sharpener 121. The first filter/mirror arrangement 116a can reflect the combined light beam 119 and cause it to travel through the beam combiner assembly window 128. The combined light beam 119 can then be directed through the reaction vessel window 122 to be incident upon the patch 102 that may be located within the reaction vessel 112. The combined light 119 being incident upon the patch 102 can induce fluorescence of chemicals within the patch 102. This may cause at least one emitted light 117a, 117b to be generated and radiate from the patch 102. The emitted light 117a, 117b be can at a certain wavelength, which may depend on the pH level and/or DO level of the environment within which the patch 102 is exposed. For example, with a combined light beam 119 comprising violet and blue light, chemicals impregnated into the patch 102 can be configured such that they emit green light 117a from the patch 102 so as to be indicative of changes in pH of a growth medium. In some embodiments, emitted light 117a of a certain wavelength (e.g., green light) can be generated when the pH level is below or above a threshold level. As another example, chemicals impregnated into the patch 102 can be configured such that emitted light 117b of a certain wavelength (e.g., red light) from the patch 102 can be generated so as to be indicative of changes in DO of a growth medium. In some embodiments, emitted red light 117b can be generated when the DO level is below or above a threshold level. Thus, a first emitted light 117a of a certain wavelength can be generated that is indicative of pH level, and a second emitted light 117b of a certain wavelength can be generated that is indicative of DO level.

Emitted light 117a, 117b from the patch 102 can travel back through the reaction vessel window 122 and further through the beam combiner assembly window 128. The emitted light 117a, 117b can be further directed to be incident upon the first filter/mirror arrangement 116a. It is contemplated for the emitted light 117a, 117b (e.g., light emitted due to fluorescence) to be generally at a wavelength that is higher than a wavelength of any of the excitation light beams 115a, 115b required to elicit the fluorescence effect. Thus, emitted light traveling 117a, 117b back toward the first filter/mirror arrangement 116a may have wavelengths that are greater than both of the first and second excitation light beams 115a, 115b. In at least one embodiment, a surface of first filter/mirror arrangement 116a can be configured to reflect the combined excitation light beams 119, but to pass emitted light beams 117a, 117b. For example, the surface of the first filter/mirror arrangement 116a can be configured to pass emitted red and/or green light coming in-through the beam combiner assembly window 128, but reflect combined violet-blue light beams so as to be directed out-through the beam combiner assembly window 128.

The emitted light 117a, 117b can be further directed to be incident upon a second filter/mirror arrangement 116b. As shown in FIG. 1A, a pass filter can be generated by both the first filter/mirror arrangement 116a and the second filter/mirror arrangement 116b. In some embodiments the pass filter can be a long pass filter. The long pass filter can be configured for passing light with certain wavelengths, or light with wavelengths greater than a minimum wavelength. For example, with the violet and blue excitation light beams 115a, 115b and the green and red emitted light beams 117a, 117b, the long pass filter may be configured to pass light with wavelengths greater than 525 nm. The long pass filter can be used by the beam combiner assembly 104 to block any light below the minimum wavelength defined by the pass filter. For example, the long pass filter can be used by the beam combiner assembly 104 to block light that is at and/or below a wavelength of the emitted light 115a, 115b. This may be done to prevent any stray excitation light 115a, 115b from passing through the long pass filter, which may be used to prevent any excitation light 115a, 115b from being detected by the detector 124. For instance, the long pass filter can be used by the beam combiner assembly 104 to block any light having a wavelength that is at and/or below 525 nm (*e.g*., block the violet and/or blue excitation light), but allow passage of any emitted light 117a, 117b (*e.g*., allow the green and/or red emitted light). Emitted light 117a, 117b can then be directed to be incident upon the detector 124. Data signals from the detector 124 may then be used to record wavelengths and/or intensities of the emitted light 117a, 117b. The recorded wavelengths and/or intensities can be associated with changes in pH and/or DO levels. For example, wavelengths at and/or near the first emitted light 117a (e.g., the green light) can be indicative of a pH level at a threshold level and/or a decreasing pH level. Wavelengths at and/or near the second emitted light 117b (e.g., the red visible light) can be indicative of DO levels at a threshold level and/or increasing DO levels. Intensities of emitted light 117a, 117b can be indicative of an amount of pH and/or DO. Recording intensities as a function of time can be used to determine or calculate rates of change of pH level and/or DO level.

While various embodiments disclose use of two excitation light beams 115a, 115b and two emitted light beams 117a, 117b, these are one exemplary. There can be any number of excitation 115 and emitted light beams 117 used. Further, the excitation 115 and emitted light beams 117 are not limited to the specific wavelengths disclosed, but the specific wavelengths are only exemplary of what can be used.

The detection system 100 can be configured so that a wavelength of an excitation light 115a beam to elicit a fluorescence response from a DO patch can be lower than a wavelength of an excitation light beam 115b to elicit a fluorescence response from a pH patch. For example, a wavelength of an excitation light beam 115a to elicit a response from a pH patch can be blue light beam (e.g., at or near 470 nm) and a wavelength of an excitation light beam 115b to elicit a response from a DO patch can be violet light beam (e.g., at or near 405 nm).

A mini-fluorometer can be used to generate excitation light beams 115a, 115b and to detect wavelengths of emitted light 117a, 117b. For example, the mini-fluorometer can be built into an integrated circuit board 134, which can be placed into communication with the computer device 110 via a hub box 118. Optoelectronics, such as a photodiode (e.g., the detector 124) for example, can be used to interrogate the patch 102 via modulation of excitation light beams 115a, 115b to detect the emitted light 117a, 117b. In some embodiments, a mini-fluorometer can be built into an integrated circuit board 134, both of which can be attached to and/or placed within the casing 126 forming the beam combiner assembly 104.

In at least one embodiment, an illumination source 114 for generating at least one excitation light beam 115 can be an LED. A band filter can be used to produce a narrow bandwidth of excitation light 115 coming from the illumination source 114. The band filter can be further used so that the gain of an LED may be adjusted to generate a desired intensity.

In an aspect, embodiments of the detection system (100) (e.g., a BioCoaster) described herein are preferably used in conjunction or combination with embodiments of a sensor insert (200) also described herein.

Referring to FIGS. 1C-1H, a typical embodiment of a beam combiner assembly 104 described herein can include a detector 124. In some embodiments, both the beam combiner assembly 104 and the detector 124 can be housed within a casing 126. The casing 126 can be configured to allow the reaction vessel 112 to be placed thereon or adjacent thereto. In some embodiments, the casing 126 can be coaster shaped. For example, the casing 126 can be a planar puck-like object, which may have a profile that is round, square, triangular, etc. In one embodiment, the casing 126 can include a rigid puck-like body with a beam combiner assembly window 128 through which excitation and/or emitted light can travel. The beam combiner assembly window 128 may include an aperture within a body of a casing 126, said aperture optionally having a transparent cover and/or lens to enable transmission of excitation 115a, 115b and/or emitted light 117a, 117b, but prevent foreign objects from entering into a casing 126. For example, the transparent cover may be a filter configured to permit certain wavelengths of light and/or bands of wavelengths to be transmitted there-through. In at least one embodiment, the beam combiner assembly window 128 can be structured with a widened aperture so as to facilitate more excitation and/or emitted light to pass there-through. Some embodiments can include a beam combiner assembly 104 with a single detector 124 or single sensing unit and some embodiments can include a plurality of detectors 124 or a plurality of sensing units.

In certain embodiments, the beam combiner assembly 104 may house illumination sources, filters, mirrors, detectors, and other electro-optics. Generation of excitation light beams 115a, 115b may be achieved through use of at least one light emitting diode ("LED"), laser, or other illumination source 114a, 114b capable of generating coherent light and/or light within a very narrow bandwidth of wavelengths. Direction of excitation light beams 115a, 115b and/or emitted light 117a, 117b within a beam combiner assembly 104 can be achieved through use of waveguides, reflectors, refractors, etc.

In certain embodiments, a BioCoaster (i.e., a detector system) described herein has one or more grooves on an upper or top side thereof. In an aspect, a flask such as one described herein, and in a preferred embodiment a wine-glass-shaped cell culture flask described herein, may have a flanged ring (or "flange") on the underside which slots or otherwise slides/slips into the one or more grooves on the upper side of a BioCoaster.

In another aspect, slotting of the flask flange into the slot on the upper side of the BioCoaster permits alignment of one or more sensors (e.g., sensor patches of sensor inserts described herein) situated inside the flask with light emitted from the upper side of the BioCoaster.

### Exemplary Cell Culturing Flasks

Described herein are embodiments of a wine-glass-shaped cell culture flask.

Embodiments of a wine-glass-shaped cell culture flask described herein may be of any size, such as 100 mL, 250 mL, 400 mL, 500 mL, 750 mL, or 1000 mL, without limitation.

In certain embodiments, the wine-glass-shaped cell culture flask is a 100 mL flask, a 250 mL flask, or a 500 mL flask. In an embodiment, the flask is 500 mL in volume.

Various embodiments of the present disclosure can be described in terms of the example shake flask 300 described herein; however, other embodiments of the shake flask 300, along with other sensor insert and BioCoaster embodiments, can be used.

In an aspect, a typical wine-glass-shaped cell culture flask described herein has a bottom which is shaped similarly to a wine glass. The wine-glass shape of the bottom portion of the flask provides increased surface area for swirling cell culture media to sweep up oxygen molecules that adhere or otherwise cling to the inner wall of the cell culture flask. Accordingly, increased oxygenation and rate of oxygenation of the cell culture media can be achieved.

Additionally, an advantage that a wine-shaped flask possesses over a cylindrical flask is that the transition of the liquid phase driven by centrifugal force of an orbital rotator in the wine-shaped flask is much smoother and produces little restriction other than friction in flowing outward and upward to the straight walls of the vessel. There are no sharp restrictions present in a wine-shaped flask; whereas a cylindrical flask possesses a disc shaped floor which can cause an abrupt merging of the solution into the vertical wall of the cylindrical vessel. The improved transition of the wine-shaped flask may also allow the operator to increase rotational speed without disturbance and agitation of the liquid phase. Increasing rotational speed shortens the cycle time and allows the liquid phase to cover more surface area faster, which can lead to an increase in the rate of oxygen transfer.

In certain embodiments, a wine-glass-shaped cell culture flask described herein has a flanged ring (or "flange") on the underside which slots or otherwise slides/slips into one or more grooves on the upper side of a BioCoaster holder. Exemplary embodiments of such a BioCoaster are described above in detail. Accordingly, cell culture flasks described herein overcome the need for flask hold-down devices.

In an aspect, any sized wine-glass-shaped cell culture flask (e.g., 125 mL, 250 mL, 400 mL, 750 mL, 500 mL, 1000 mL, etc.) described herein may be fitted with a flange and used with the same BioCoaster holder.

In another aspect, slotting of the flask flange into the slot on the upper side of the BioCoaster permits alignment of one or more sensors (e.g., sensor patches of sensor inserts described herein) situated inside the flask with light emitted from the upper side of the BioCoaster.

In an aspect, a wine-glass-shaped cell culture flask described herein may be produced by any conventional or known method of manufacture, including mass production methods such as injection or blow molding.

In an aspect, an upper portion of a wine-glass-shaped cell culture flask described herein is a sloping wall. In certain embodiments, the upper portion of a wine-glass-shaped cell culture flask described herein is of the same or substantially the same angle as a traditional cell culture flask.

In certain preferred embodiments, the upper portion of a wine-glass-shaped cell culture flask described herein is of a lesser angle than a traditional cell culture flask. By decreasing the angle of the walls of an upper portion of the flask, easier removal of built-up carbon dioxide from the flask head-space is possible.

In certain embodiments, a wine-glass-shaped cell culture flask described herein may be fitted with any known flask stopper, including rubber stoppers, cork stoppers.

In other embodiments, a wine-glass-shaped cell culture flask described herein may have a flask mouth with standard threading such that the flask may be fitted with any known flask cap having standard threads.

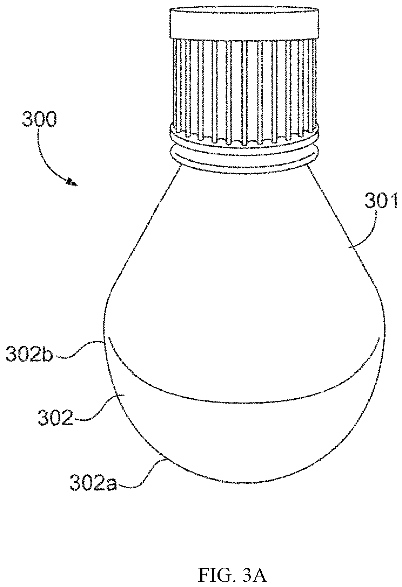

Referring to FIG. 3A, a typical embodiment of a wine-glass-shaped cell culture flask 300 described herein has an upper portion 301 having angled walls which are of a lesser angle than the walls of a conventional shake flask, and a lower portion 302 which is substantially similar to the shape of a wine glass.

By "shape of a wine glass," it is meant that the lower portion 302 of a wine-glass-shaped cell culture flask 300 described herein has a shape which is flat on the bottom, curving upward in a curved bottom wall 302a to a vertical or substantially vertical wall 302b extending upward to the upper portion 301 of the flask.

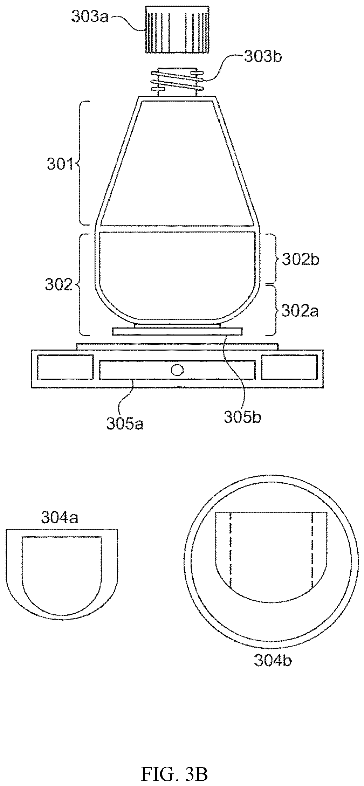

Again referring to FIG. 3B, a typical embodiment of a wine-glass-shaped cell culture flask 300 described herein may be fitted with any conventional flask stopper or cap 303a and the flask mouth may be threaded 303b to fit a flask cap having standard threading, for example. Furthermore, the wine-glass-shaped cell culture flask may have an insert flange 304a on its underside which, when slotted 304b into a flask-flange hold-down slot 305b situated on the upper side of a BioCoaster 305a, secures the flask without the need of flask hold-down devices.

### Exemplary Inverted Shake Flask Systems

Also described herein are an inverted shake flask system and method of culturing cells using the same.

The shake flask system can comprise a shake flask of any size, such as 100 mL, 250 mL, 500 mL, 750 mL, or 1000 mL, for example and without limitation. In certain embodiments, the shake flask is a 100 mL shake flask, a 250 mL shake flask, or a 500 mL shake flask.

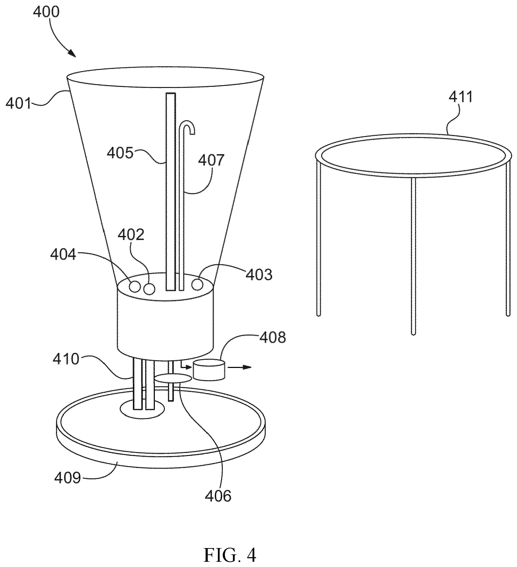

Referring to FIG. 4, the inverted shake flask system 401 comprises a shake flask 401 oriented in an inverted fashion, the shake flask having a stopper 402, the stopper housing a light scattering source 403 and one or more optical sensors 404. Additionally, the inverted shake flask system 401 comprises an inlet 405 for air or other gas to enter the shake flask and an air filter 406 connected to said inlet 405, and further comprises an outlet 407 connected to a vacuum pump 408 to permit air or other gas to exit the shake flask. Additionally, a detector system (e.g., a BioCoaster described above) 409 is in communication with the one or more optical sensors 404 via fiber optics 410. Furthermore, a stand or holder 411 is preferably used to maintain the inverted shake flask 401 in an upright orientation.

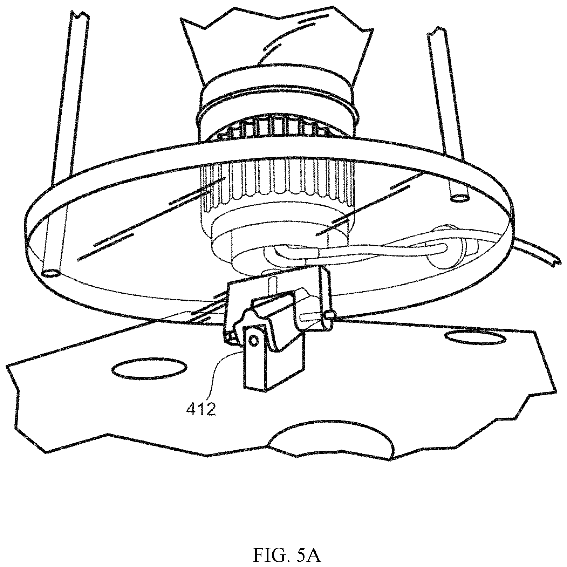

In an aspect, an inverted shake flask system described herein may use a swivel adapter 412. FIGs. 5A-5E depict an inverted flask 401 attached to a swivel adapter 412. The use of a swivel adapter 412 allows a swirling pattern 413 to occur inside the inverted flask. This swirling pattern 413 can mimic the pattern observed when a flask is swirled by a wrist action. FIGs. 5D-5E demonstrate that the inverted flask can be mixed at speeds ranging from 100 rpm to 150 rpm. These speeds represent examples of how fast the inverted flask can spin and are not in any way limiting to this aspect. Accordingly, increased oxygenation and rate of oxygenation of the cell culture media can be achieved.

In an aspect, an inverted shake flask system described herein may use any type of flask, including traditional flasks (e.g., Erlenmeyer flasks) and wine-glass-shaped flasks described herein.

In an aspect, any conventional or commercially available vacuum system may be used in conjunction or combination with the inverted shake flask system, such as a mini vacuum system.

In an aspect, any conventional or commercially available air filter may be used in conjunction or combination with the inverted shake flask system.

In an aspect, a stopper used in the inverted shake flask system described herein may be made of any suitable material, such as a rubber stopper.

In a preferred embodiment, a stopper system is employed. By "stopper system" what is meant is a combination in which the stopper houses one or more optical sensors in operable communication with a BioCoaster by, for example and without limitation, fiber optics, and which houses one or more light scattering sources.

In another preferred embodiment, a stopper system described herein houses one or more optical sensors in operable communication with a BioCoaster by, for example and without limitation, fiber optics, and which houses one or more light scattering sources, and which permits air/gas such as oxygen and/or carbon dioxide to enter via an inlet and exit via an outlet, optionally connected to a vacuum pump.

In a preferred embodiment, the outlet is connected to a vacuum pump.

In an aspect, the light scattering source may be either infrared or visible light.

In a preferred embodiment, the light scattering source is infrared. The light scatter source emits light into the vessel and the detector detects scattered light. It is contemplated for the light scattering source to be an infrared light scattering source because as the cells grow they scatter more infrared. Thus, the amount of scattered infrared light present (or detected) is directly related to the amount of cell growth. This provides a means to monitor cell growth. In addition, infrared light facilitates analysis with only approximately 35 mm of liquid depth, whereas if use of visible light is used the amount of liquid depth required would be approximately 60 mm.

In some embodiments, the system comprises a rotation unit or shaking unit configured to rotate or shake the reaction vessel to further enhance cell growth.

It has been observed that, by employing a method or system described herein with an inverted cell culture flask, deficiencies of existing shake-flask systems and methods can be overcome, including poor oxygenation of culture media and difficulty of removing carbon dioxide from the reaction vessel (i.e., flask).

Additionally, an advantage that an inverted-flask possesses over a cylindrical flask is that the transition of the liquid phase driven by centrifugal force of an orbital rotator in the inverted-shaped flask is much smoother and produces little restriction other than friction in flowing outward and upward to the straight walls of the vessel. There are no sharp restrictions present in a inverted-shaped flask; whereas a cylindrical flask possesses a disc shaped floor which can cause an abrupt merging of the solution into the vertical wall of the cylindrical vessel. The improved transition of the inverted-shaped flask may also allow the operator to increase rotational speed without disturbance and agitation of the liquid phase. Increasing rotational speed shortens the cycle time and allows the liquid phase to cover more surface area faster, which can lead to an increase in the rate of oxygen transfer.

Thus, systems and methods described herein provide a means of culturing cells wherein the problem of inhibiting gas (e.g., air or oxygen) flow is eliminated. Flasks, systems, and methods described herein permit air/oxygen to freely flow regardless of the size of the flask neck or the type of material used in the flask stopper.

Moreover, embodiments of the flasks, systems and methods described herein enable the use of a faster rotation without the concern of wetting a flask stopper.

Various embodiments of the present disclosure can be described in terms of the example inverted shake flask system described herein; however, other embodiments of the inverted shake flask system, can be used. For example, other embodiments of the inverted shake flask system incorporate other embodiments of the sensor insert, BioCoaster, and/or flask.

It will be apparent to those skilled in the art that numerous modifications and variations of the described examples and embodiments are possible in light of the above teachings of the disclosure. The disclosed examples and embodiments are presented for purposes of illustration only. Other alternate embodiments may include some or all of the features disclosed herein. Therefore, it is the intent to cover all such modifications and alternate embodiments as may come within the true scope of this invention, which is to be given the full breadth thereof. Additionally, the disclosure of a range of values is a disclosure of every numerical value within that range, including the end points.

## Claims

1. An inverted cell culture flask system for culturing cells comprising:
a flask oriented in an inverted fashion,
a vacuum pump,
a stopper,
a light scattering source,
a detector system, and
at least one optical sensor operably connected to the detector system.

2. The system according to claim 1, wherein the light scattering source scatters infrared or visible light.

3. The system according to claims 1-2, wherein the stopper houses at least one of:
the light scattering source, and
the at least one optical sensor operably connected to the detector system.

4. The system according to claim 1-3, further comprising a rotation unit or shaking unit configured to rotate or shake the flask.

5. A method of culturing cells comprising culturing cells in a flask oriented in an inverted fashion, wherein the flask comprises a stopper, the stopper comprising a light scattering source and one or more optical sensors, operably connected to a detector system.

6. The method according to claim 7, wherein the light scattering source scatters infrared or visible light.

7. The method according to claim 6, further comprising evacuating air/gas from the flask via an outlet connected to a vacuum pump.

8. The method according to claims 6-7, further comprising rotating or shaking the flask.

9. A sensor insert comprising:
at or proximate to a first end, a hollow or substantially hollow tube portion,
a space for housing one or more carbon dioxide absorbent substances,
an extension length, and
at a second end, a sensor tab configured to hold one or more sensor patches.

10. The sensor insert according to claim 9, wherein the one or more sensor patches is a dissolved oxygen sensor or a pH sensor.

11. The sensor insert according to claims 9-10, wherein the one or more sensor patches comprise a dissolved oxygen sensor and a pH sensor.

12. The sensor insert according to claims 9-11, further comprising one or more vents.

13. The sensor insert according to claims 9-12, wherein the sensor patches are configured to operably communicate with a detector system.

14. A cell culture flask comprising a lower portion and an upper portion,
said lower portion comprising a flat bottom and a curved wall portion curving upward to a vertical or substantially vertical wall portion extending upward to the upper portion,
said upper portion comprising an angled wall, angled upward to a flask neck or mouth.

15. A flask stopper comprising a light scattering source and one or more optical sensors.
